# EUROPEAN PATENT APPLICATION

(11) **EP 3 872 166 A1**
(43) Date of publication of application: **01.09.2021**
(21) Application number: 19876360.9
(22) Date of filing: 24.10.2019
(51) Int. Cl.: C12N 1/02, C12N 1/14, C12N 1/20, C12Q 1/04, C12Q 1/6851, C12Q 1/689

(54) **CELL COLLECTION METHOD**

(30) Priority: 25.10.2018 JP 2018200476
(71) Applicant: Nissui Pharmaceutical Co., Ltd., Tokyo 110-8736 (JP)
(72) Inventor: YANAGIDA, Risa, Yuki-shi, Ibaraki 307-0036 (JP)
(74) Representative: Hartz, Nikolai
(86) International application number: PCT/JP2019/041658
(87) International publication number: WO 2020/085420

(57) **Abstract**

To provide a means for collecting microorganism cells simply and with high efficiency.

A method for collecting cells of one or more microorganisms selected from the group consisting of gram-positive bacteria (excluding mycoplasma), gram-negative bacteria, and fungi in a specimen, the method comprising adding one or more proteins selected from the group consisting of albumin, casein, hydrolyzed casein, milk protein, and gelatin to the specimen and collecting a resulting flocculate.

SEQUENCE LISTING

<110>  Nissui Pharmaceutical Co., Ltd.

<120>  Method for Collecting Bacteria

<130>  NSP0015

<150>  JP2018-200476
<151>  2018-10-25

<160>  3

<170>  PatentIn version 3.5

<210>  1
<211>  20
<212>  DNA
<213>  artificial sequence

<220>
<223>  synthetic oligonucleotide

<400>  1
agaatttgat cmtggctcag                                                   20


<210>  2
<211>  20
<212>  DNA
<213>  artificial sequence

<220>
<223>  synthetic oligonucleotide

<400>  2
ctttacgccc artrawtccg                                                   20


<210>  3
<211>  22
<212>  DNA
<213>  artificial sequence

<220>
<223>  synthetic oligonucleotide


<220>
<221>  misc_feature
<222>  (1)..(1)
<223>  6FAM-dt

<220>
<221>  misc_feature
<222>  (2)..(2)
<223>  n is a, c, g, or t

<220>
<221>  misc_feature
<222>  (22)..(22)
<223>  dg-BHQ

<400> 3
nnttaccgcg gctgctggca cn 22

## Description

### Technical Field

The present invention relates to a method for collecting microorganism cells.

### Background Art

Contamination with microorganisms is one of important management indicators of food and drink, medicine, and reagents used in, for example, regenerative medicine. Accordingly, methods for avoiding contamination with microorganisms through sterilization using an autoclave or a filter are known. However, when such sterilization is not applicable, it is necessary to treat them aseptically and to finally examine for microbial contamination. In addition, even if sterilization treatment has been conducted, it is necessary to verify whether the effect of the sterilization treatment has been sufficient or not. However, if a small amount of extremely small microorganisms is contaminated, it is difficult to detect them with a high accuracy. In addition, when a specimen contains a large volume, it is also impractical to examine the whole specimen. Accordingly, methods for efficiently collecting or separating microorganisms have been considered.

For example, as a method for simply and highly efficiently collecting cells, a method of using a protein called MDP1, which binds to sugars on cell surfaces, as a flocculation accelerator to precipitate cells without performing centrifugation. In addition, a method of adding a solid support, such as particles conjugated with an antibody, has been reported (e.g., see Patent Literature 1).

In addition, a method of flocculating bacteria by adding a microbial extract protein and adjusting the pH of a cell suspension to 1 to 5 and separating the bacterial cells from the bacterial suspension is known (e.g., see Patent Literature 2).

However, the method of Patent Literature 1 uses MDP1, a protein of Mycobacterium bovis (BCG), and the method problematically involves a complicated process of purifying MDP1. In addition, in the method of Patent Literature 2, microbial extract proteins include not only various proteins (including enzymes, such as DNase and RNase) but also contaminants, such as DNA and RNA, and therefore there is a risk of influencing the test of microorganisms after the cell collection. Furthermore, it was burdensome to involve the need of adjusting the pH of the cell suspension.

From the above, a method for collecting microorganism cells with high efficiency and without performing a complicated process has been hoped for.

### Citation List

### Patent Literatures

Patent Literature 1: JP-A-2010-104250
Patent Literature 2: JP-A-sho 50-135275

### Summary of Invention

### Technical Problem

Accordingly, the present invention aims to provide a method for collecting microorganism cells with a simple process and with high efficiency.

### Solution to Problem

In view of such circumstances, the present inventor has made energetic studies to solve the above problems and, as a result, has found that cells of one or more microorganisms selected from the group consisting of gram-positive bacteria, gram-negative bacteria, and fungi can be collected as a flocculate with high efficiency by adding a protein selected from specific five proteins that are easily available and have known structures and preferably further adding a water-insoluble support to a specimen, and has accomplished the present invention.

[1] A method for collecting cells of one or more microorganisms selected from the group consisting of gram-positive bacteria (excluding mycoplasma), gram-negative bacteria, and fungi in a specimen, the method comprising adding one or more proteins selected from the group consisting of albumin, casein, hydrolyzed casein, milk protein, and gelatin to the specimen and collecting a resulting flocculate.
[2] The method according to [1], wherein the method collects cells of gram-positive bacteria (excluding mycoplasma) and/or gram-negative bacteria.
[3] The method according to [1] or [2], wherein the specimen after addition of the protein has a pH of above 5.0 and 11.0 or less.
[4] The method according to any one of [1] to [3], wherein the specimen is a liquid specimen selected from the group consisting of sterilized water, physiological saline, a liquid culture medium, a biological sample, a culture supernatant, a buffer solution, and a Ringer's solution.
[5] The method according to any one of [1] to [4], wherein the method collects cells of gram-positive bacteria.
[6] The method according to any one of [1] to [5], wherein the gram-positive bacteria are one or more selected from the group consisting of Staphylococcus, Enterococcus, Streptococcus, Clostridium, Corynebacterium, and Bacillus.
[7] The method according to any one of [1] to [5], wherein the gram-positive bacteria are one or more selected from the group consisting of Staphylococcus aureus, Bacillus subtilis, and Clostridium sporogenes.
[8] The method according to any one of [1] to [7], wherein a water-insoluble support is further added to the specimen.
[9] A method for measuring one or more microorganisms selected from the group consisting of gram-positive bacteria, gram-negative bacteria, and fungi, the method comprising subjecting cells of the one or more microorganisms selected from the group consisting of gram-positive bacteria, gram-negative bacteria, and fungi collected by the method according to any one of [1] to [8] to a polymerase chain reaction.
[10] A method for measuring one or more microorganisms selected from the group consisting of gram-positive bacteria, gram-negative bacteria, and fungi, the method comprising enrichment of cells of the one or more microorganisms selected from the group consisting of gram-positive bacteria, gram-negative bacteria, and fungi collected by the method according to any one of [1] to [8].

### Advantageous Effects of Invention

The method of the present invention can collect cells of one or more microorganisms selected from the group consisting of gram-positive bacteria, gram-negative bacteria, and fungi by adding a protein selected from specific known five proteins that are easily available with extremely high efficiency compared to when the protein is not added. In addition, when the method of the present invention is used, cells of one or more microorganisms selected from the group consisting of gram-positive bacteria, gram-negative bacteria, and fungi, which are reference strains for validation in the Sterility Test prescribed in paragraph 4.06 of The Japanese Pharmacopoeia (Seventeenth Edition), can be efficiently collected from a specimen.

The method of the present invention does not require to prepare microorganisms according to the test date and to prepare microorganism proteins or microbial extract proteins and does not require to verify whether the solvent and reagent necessary for preparing them are contaminated with one or more microorganisms selected from the group consisting of gram-positive bacteria, gram-negative bacteria, and fungi contained into, either. Accordingly, the method is highly convenient.

The method of the present invention optionally uses a water-insoluble support, such as latex particles, and thereby the flocculate after separation is unlikely to diffuse and also can be easily visually observed, even if the cells of one or more microorganisms selected from the group consisting of gram-positive bacteria, gram-negative bacteria, and fungi are in a small amount. Accordingly, the supernatant after precipitation of the bacteria by cell collection can be easily removed.

The method of the present invention has a low risk of damaging bacteria and therefore can culture the collected bacterial cells. Consequently, a colony of the cultured cells can also be subjected to another test.

In addition, in the method of the present invention, since no contamination with DNA, RNA, DNase, RNase, and other proteins derived from cells is caused, it is easy to perform a nucleic acid amplification reaction procedure after the cell collection.

### Description of Embodiments

Hereinafter, examples of embodiments of the present invention will be described, but the present invention is not limited to these embodiments at all.

In the present invention, the phrase "collection of cells of one or more microorganisms selected from the group consisting of gram-positive bacteria, gram-negative bacteria, and fungi" means that one or more microorganisms selected from the group consisting of gram-positive bacteria, gram-negative bacteria, and fungi contained in a specimen are separated and concentrated. When cells of one or more microorganisms selected from the group consisting of gram-positive bacteria, gram-negative bacteria, and fungi are collected, the subsequent sterility test can be easily conducted.

The method for collecting cells of one or more microorganisms selected from the group consisting of gram-positive bacteria, gram-negative bacteria, and fungi of the present invention collects the flocculate formed by adding one or more proteins selected from the group consisting of albumin, casein, hydrolyzed casein, milk protein, and gelatin to a specimen.

In the present invention, the gram-positive bacteria are bacteria that are stained dark blue or deep purple by gram staining. The gram-positive bacteria are generally characterized by a thick cell wall, no outer membrane, and a thick peptidoglycan layer. The gram-positive bacteria are classified into gram-positive cocci and gram-positive bacilli in accordance with the shapes. Mycoplasma is classified as a gram-positive bacterium for convenience, but is excluded from the gram-positive bacteria in the present invention, because mycoplasma does not have a cell wall and cannot be gram-stained.

Examples of the gram-positive cocci include, but not limited to, Staphylococcus (e.g., Staphylococcus aureus), Enterococcus (e.g., Enterococcus faecalis), and Streptococcus (e.g., Streptococcus pneumoniae).

Examples of the gram-positive bacilli include, but not limited to, Clostridium (e.g., Clostridium sporogenes), Corynebacterium (e.g., Corynebacterium renale), and Bacillus (e.g., Bacillus subtilis).

Among these gram-positive bacteria, the subject is preferably one or more selected from the group consisting of Staphylococcus aureus, Bacillus subtilis, and Clostridium sporogenes. These bacteria are reference strains for validation in the Sterility Test prescribed in paragraph 4.06 of The Japanese Pharmacopoeia (Seventeenth Edition). Implementing the cell collection method of the present invention serves to secure the sterility of a sample to be used in the sterility test.

In the present invention, the gram-negative bacteria are bacteria that do not retain the crystal violet stain in gram-staining. The gram-negative bacteria are generally characterized by outer membrane covered by lipopolysaccharides and a cell wall with a thin peptidoglycan layer. The gram-negative bacteria are classified into gram-negative cocci and gram-negative bacilli in accordance with the shapes.

Examples of the gram-negative cocci include, but not limited to, Neisseria (e.g., Neisseria meningitis) and Veillonella (e.g., Veillonella parvula).

Examples of the gram-negative bacilli include, but not limited to, Escherichia (e.g., Escherichia coli), Salmonella (e.g., Salmonella enteritidis), Enterobacter (e.g., Enterobacter cloacae and Enterobacter aerogenes), Bacteroides (e.g., Bacteroides fragilis), and Pseudomonas (e.g., Pseudomonas aeruginosa, Pseudomonas cepacia, and Pseudomonas putida).

Examples of the fungi in the present invention include, but not limited to, Candida (e.g., Candida albicans) and Aspergillus (e.g., Aspergillus brasiliensis).

In the present invention, cells of gram-negative bacteria and fungi can be collected even without addition of a protein, but the method in which the protein is added of the present invention can improve the cell collection efficiency. Accordingly, the method of the present invention can collect cells of Staphylococcus aureus, Bacillus subtilis, Clostridium sporogenes, Pseudomonas aeruginosa, Candida albicans, and Aspergillus brasiliensis, which are reference strains for validation in the sterility test. Therefore, the cell collection method of the present invention can be used to secure the sterility of such samples to be used in the sterility test.

In the present invention, the specimen is a sample to be tested for infection with one or more microorganisms selected from the group consisting of gram-positive bacteria, gram-negative bacteria, and fungi and is preferably liquid. Examples of the specimen include water (such as sterilized water and distilled water), physiological saline, a liquid culture medium (for example, a medium that is used for culturing cells such as eukaryotic cells, such as RPMI1640, DMEM, α-MEM, and HANKS), a biological sample, a culture supernatant, a buffer solution (for example, Tris-HCl buffer, HEPES buffer, MOPS buffer, HEPPS buffer, TAPS buffer, and phosphate buffer (PBS) each adjusted to a predetermined pH), and a ringer solution. The specimen may also be a cell suspension supernatant or a culture supernatant after cell culture.

The protein to be used in the present invention is one or more selected from the group consisting of albumin (for example, animal albumin, such as bovine serum albumin (BSA), human serum albumin, primate serum albumin, rabbit serum albumin, rodent serum albumin, horse serum albumin, goat serum albumin, sheep serum albumin, canine serum albumin, guinea pig serum albumin, chicken serum albumin, and pig serum albumin), casein, hydrolyzed casein, milk protein (for example, trade name: Block Ace (manufactured by DS Pharma Biomedical Co., Ltd.)), and gelatin. From the viewpoint of avoiding contamination of bacteria when the collected cells of one or more microorganisms selected from the group consisting of gram-positive bacteria, gram-negative bacteria, and fungi are subjected to PCR (polymerase chain reaction), it is preferable to use sterile protein. These proteins can be used singly or in combination of two or more.

In the method for collecting cells of one or more microorganisms selected from the group consisting of gram-positive bacteria, gram-negative bacteria, and fungi of the present invention, a flocculate obtained by adding the above-mentioned protein to a specimen is collected. Specifically, a specimen and the above-mentioned protein are put in a centrifugation tube. In order to cause flocculation without fail, the tube may be stirred or left for an arbitrary time (for example, for 1 second to 60 minutes). The resulting flocculate can be collected by, for example, centrifugation. The centrifugal force in the centrifugation is not particularly limited and is, for example, 3,000 to 30,000 G, preferably 5,000 to 28,000 G, and more preferably 14,000 to 25,000 G. The centrifugation time is not particularly limited and is, for example, from 10 seconds to 120 minutes, preferably from 1 minute to 60 minutes, and more preferably from 10 minutes to 30 minutes. The temperature of the centrifugation is not particularly limited and is, for example, from 4°C to 35°C, preferably from 7°C to 30°C, and more preferably from 10°C to 25°C. The flocculate of one or more microorganisms selected from the group consisting of gram-positive bacteria, gram-negative bacteria, and fungi is precipitated at the bottom of the centrifugation tube by the centrifugation. The supernatant is then removed to obtain the flocculate of one or more microorganisms selected from the group consisting of gram-positive bacteria, gram-negative bacteria, and fungi as a residue. As needed, the centrifugation and the step of removing the supernatant may be repeated twice or more.

In order to obtain the flocculate, a specimen may be optionally subjected to physiological treatment and/or chemical treatment. Examples of the physiological treatment include heating treatment, ultrasonication treatment, and freezing and thawing treatment. Examples of the chemical treatment include chemical reagent treatment, for example, denaturation treatment using a digestive enzyme, a surfactant, a lysis agent, or the like.

The amount of the protein to be used is not particularly limited as long as the amount allows to sufficiently flocculate one or more microorganisms selected from the group consisting of gram-positive bacteria, gram-negative bacteria, and fungi, and is, for example, preferably from 0.1 to 500 mg, more preferably from 0.2 to 100 mg, and further preferably from 2 to 20 mg based on 1 mL of the specimen.

The pH of the specimen after addition of the protein is not particularly limited. When the purpose is to obtain live bacteria, the pH is within a range in which one or more microorganisms selected from the group consisting of gram-positive bacteria, gram-negative bacteria, and fungi is not denatured, and is, for example, preferably above 5.0 and 11.0 or less, more preferably from 5.1 to 10.0, further preferably in a range of from 6.0 to 8.0, and even more preferably in a range of from 6.8 to 7.4. In addition, addition of a protein and collection of the flocculate of one or more microorganisms selected from the group consisting of gram-positive bacteria, gram-negative bacteria, and fungi can be conducted under conditions of, for example, from 4°C to 35°C, preferably from 7°C to 30°C, and more preferably from 10°C to 25°C.

In the present invention, in addition to the protein, a water-insoluble support can be added. The water-insoluble support is not particularly limited as long as it is possible to flocculate one or more microorganisms selected from the group consisting of gram-positive bacteria, gram-negative bacteria, and fungi when used together with the protein. Examples of the water-insoluble support include latex particles (e.g., organic polymer latex particles) such as polystyrene, a styrene-methacrylic acid copolymer, a styrene-glycidyl(meth)acrylate copolymer, a styrene-styrenesulfonate copolymer, a methacrylic acid polymer, an acrylic acid polymer, an acrylonitrile butadiene styrene copolymer, a vinyl chloride-acrylic acid ester copolymer, and a poly(vinyl acetate-acrylate); latex particles processed for physical adsorption or processed with chemical bonding through a carboxyl group; colored cellulose particles, silica particles, and gold colloid particles. Among these supports, latex particles, in particular, organic polymer latex particles are preferable from the point of the flocculating property of one or more microorganisms selected from the group consisting of gram-positive bacteria, gram-negative bacteria, and fungi. Since the flocculate of bacterial cells after centrifugation can be visualized by adding a water-insoluble support, it is possible that the bacterial cells are prevented from being removed when the supernatant is removed.

The shape of the water-insoluble support is not particularly limited and is spherical or substantially spherical from the viewpoint of sufficiently flocculating one or more microorganisms selected from the group consisting of gram-positive bacteria, gram-negative bacteria, and fungi.

The particle diameter of the water-insoluble support is not particularly limited, and the average particle diameter is, for example, preferably from 10 to 2,000 nm, more preferably from 50 to 1,500 nm, and further preferably from 100 to 1,000 nm from the points of visibility and simplicity of experiments. The particle diameter of the water-insoluble support can be measured by, for example, transmission electron microscopy (TEM).

The amount of the water-insoluble support to be used is not particularly limited as long as the amount allows to sufficiently flocculate one or more microorganisms selected from the group consisting of gram-positive bacteria, gram-negative bacteria, and fungi, and is, for example, from 0.0005 to 10 mg, preferably from 0.001 to 1 mg, and further preferably from 0.002 to 0.1 mg based on 1 mL of the specimen.

The weight ratio of the protein to the water-insoluble support, (the protein) : (water-insoluble support), is preferably from 1 : 1 to 50,000 : 1, more preferably from 10 : 1 to 10,000 : 1, further preferably from 200 : 1 to 1,000 : 1 from the point of the flocculating property of one or more microorganisms selected from the group consisting of gram-positive bacteria, gram-negative bacteria, and fungi.

The present invention also relates to a method for measuring one or more microorganisms selected from the group consisting of gram-positive bacteria, gram-negative bacteria, and fungi by subjecting the residue containing the one or more microorganisms selected from the group consisting of gram-positive bacteria, gram-negative bacteria, and fungi collected by the above-described method to a polymerase chain reaction (PCR).

The method can perform a detection test of one or more microorganisms selected from the group consisting of gram-positive bacteria, gram-negative bacteria, and fungi using the residue containing a flocculate of the one or more microorganisms selected from the group consisting of gram-positive bacteria, gram-negative bacteria, and fungi obtained by the above-described method by a known PCR method or a modification thereof (e.g., Real-Time PCR) under known conditions by performing DNA extraction through a known method with a commercially available kit (e.g., a bacterial DNA extraction kit (manufactured by Mitsui Chemicals, Inc.)). On this occasion, one or more microorganisms selected from the group consisting of gram-positive bacteria, gram-negative bacteria, and fungi can be detected using a primer and a probe targeting a region conserved in the one or more microorganisms selected from the group consisting of gram-positive bacteria, gram-negative bacteria, and fungi, and one or more microorganisms selected from the group consisting of gram-positive bacteria, gram-negative bacteria, and fungi can also be identified using a primer and a probe targeting a region specific to the microorganism to be detected.

The present invention also relates to a kit for collecting cells of one or more microorganisms selected from the group consisting of gram-positive bacteria, gram-negative bacteria, and fungi, the kit including the protein for a specimen and preferably further a water-insoluble support. The protein and the water-insoluble support contained in the kit are as described above. The kit may appropriately include a manual describing the procedure described above.

Since the present invention can further collect cells of one or more microorganisms selected from the group consisting of gram-positive bacteria, gram-negative bacteria, and fungi as the target of cell collection while retaining the proliferative capacity, the collected cells of one or more microorganisms selected from the group consisting of gram-positive bacteria, gram-negative bacteria, and fungi can also be used in various tests. For example, the collected cells of one or more microorganisms selected from the group consisting of gram-positive bacteria, gram-negative bacteria, and fungi are inoculated, for example, on a plate medium or in a liquid culture medium for enrichment, and the collected cells of one or more microorganisms selected from the group consisting of gram-positive bacteria, gram-negative bacteria, and fungi can also be used for, for example, measurement of the number of the cells, identification tests such as biochemical examination, or reagent sensitivity test.

### Examples

The present invention will now be described in further detail by means of Examples, but is not limited to these Examples in any way.

### Example 1: Effect of cell collection by protein and water-insoluble support

Staphylococcus aureus (S. aureus) cells precultured using a sheep blood agar medium (trade name: Nissui Plate Sheep Blood Agar, manufactured by Nissui Pharmaceutical Co., Ltd.) were suspended in physiological saline to prepare a bacterial suspension with a turbidity MacFarland of 1.0. The bacterial suspension was then diluted 10³-fold to prepare a diluted bacterial liquid, and 0.1 mL of the diluted bacterial liquid was added to 24.9 mL of physiological saline. To this were added 350 µL of 30% (w/v) BSA solution (bovine serum-derived BSA solution, fatty acid free, manufactured by FUJIFILM Wako Pure Chemical Corporation) and 2.5 µL of 10% (w/v) latex solution (polystyrene latex particles having a particle diameter of 315 nm (IMMUTEX (registered trademark, manufactured by JSR Life Sciences Corporation))). The solutions were stirred, followed by centrifugation (20,000 G, 15 minutes, 10°C), and the supernatant was removed to obtain 1 mL of a test liquid. This test liquid was diluted 10-fold, and 10 µL of the resulting bacterial liquid was inoculated on a sheep blood agar medium (the same as above), followed by culturing overnight (37°C, aerobic condition). As a comparison control not performing cell collection, the bacterial suspension was diluted 10⁶-fold, and 100 µL thereof was inoculated on a sheep blood agar medium (the same as above).

DNA was extracted from the test liquid using a bacterial DNA extraction kit (manufactured by Mitsui Chemicals, Inc.), and real time PCR was conducted using Yeast-made Taq DNA polymerase (manufactured by Mitsui Chemicals, Inc.), forward primer: agagtttgatcMtggctcag (SEQ ID NO: 1), reverse primer: ctttacgcccaRtRaWtccg (SEQ ID NO: 2), and probe: 6FAM-tNttaccgcggctgctggcacg-BHQ (SEQ ID NO: 3). The reaction was carried out at 95°C for 5 seconds followed by 45 cycles at 95°C for 5 seconds and 60°C for 30 seconds as one cycle.

Table 1 shows the results of the culture test.

**[Table 1]**

| cell | cell collection | number of colony | | | recovery rate |
|---|---|---|---|---|---|
| | | test 1 | test 2 | average | |
| S. aureus | BSA + latex | 21 | 28 | 24.5 | 73% |
| | BSA | 30 | 30 | 30 | 90% |
| | latex | 2 | 2 | 2 | 6% |
| | saline | 2 | 1 | 1.5 | 4% |
| | cell collection was not conducted | 40 | 27 | 33.5 | - |

The results of Table 1 demonstrated that when BSA was added alone and when BSA and latex were added in combination, the number of colonies was close to that obtained when cell collection was not conducted, as compared to those obtained when the latex was added alone and when physiological saline was added. Accordingly, cells of Staphylococcus aureus, which is a gram-positive bacterium, can be efficiently collected by adding BSA. Furthermore, the precipitate by centrifugation was visualized by adding latex in addition to BSA, and the supernatant was consequently easily removed.

Table 2 shows the results of PCR.

**[Table 2]**

| cell | cell collection | Cq value | | |
|---|---|---|---|---|
| | | test 1 | test 2 | average |
| S. aureus | BSA + latex | 31.03 | 31 | 31.015 |
| | BSA | 30.56 | 30.23 | 30.395 |
| | latex | 32.46 | 35.6 | 34.03 |
| | saline | 37.28 | 33.73 | 35.505 |
| | cell collection was not conducted | 31.01 | NT | 31.01 |

The results of Table 2 demonstrated that also in PCR, when BSA was added alone and when BSA and latex were added in combination, the Cq value was close to that obtained when cell collection was not conducted. Accordingly, cells of Staphylococcus aureus, which is a gram-positive bacterium, can be sufficiently collected by performing addition of BSA and concentration. In contrast, when physiological saline was added and when latex was added alone, the Cq value increased, and the cell collection was thus insufficient.

### Example 2: Relationship between gram-stainability and cell collection effect

Corynebacterium renale (C. renale), Neisseria meningitis (N. meningitis), and Escherichia coli (E. coli) cells were precultured using a sheep blood agar medium (trade name: Nissui Plate Sheep Blood Agar, manufactured by Nissui Pharmaceutical Co., Ltd.) or a chocolate agar medium (trade name: Nissui Plate Chocolate Agar EX II, manufactured by Nissui Pharmaceutical Co., Ltd.) were each suspended in physiological saline to prepare bacterial suspensions with a turbidity MacFarland of 1.0. The bacterial suspensions were then each diluted 10³-fold to prepare respective diluted bacterial liquids, and 0.1 mL of each of the diluted bacterial liquids was added to 24.9 mL of physiological saline. To this added was 350 µL of 30% (w/v) BSA solution (fatty acid free, manufactured by FUJIFILM Wako Pure Chemical Corporation). The solutions were stirred, followed by centrifugation (20,000 G, 15 minutes, 10°C), and the supernatant was removed to obtain 1 mL of each test liquid. These test liquids (excluding the test liquid of Corynebacterium renale) were diluted 10-fold, and the resulting bacterial liquids and the test liquid of Corynebacterium renale were each inoculated on a sheep blood agar medium (the same as above) or on a chocolate agar medium (the same as above), followed by culturing overnight (37°C, aerobic condition). On this occasion, Neisseria meningitis was cultured using AnaeroPack-CO2 (manufactured by SUGIYAMA-GEN Co., Ltd.) under a condition of 37°C. As a comparison control not performing cell collection, the bacterial suspension was diluted 10⁶-fold, and 100 µL thereof was inoculated on a sheep blood agar medium (the same as above).

DNA was extracted from the test liquid using a bacterial DNA extraction kit (manufactured by Mitsui Chemicals, Inc.), and real time PCR was conducted using Yeast-made Taq DNA polymerase (manufactured by Mitsui Chemicals, Inc.), forward primer: agagtttgatcMtggctcag (SEQ ID NO: 1), reverse primer: ctttacgcccaRtRaWtccg (SEQ ID NO: 2), and probe: 6FAM-tNttaccgcggctgctggcacg-BHQ (SEQ ID NO: 3). The reaction was carried out at 95°C for 5 seconds followed by 45 cycles at 95°C for 5 seconds and 60°C for 30 seconds as one cycle.

Table 3 shows the results of the culture test.

**[Table 3]**

| cell | cell collection | number of colony | | | recovery |
|---|---|---|---|---|---|
| | | test 1 | test 2 | average | rate |
| C. renale | BSA | 96 | 84 | 90 | 68% |
| | saline | 0 | 0 | 0 | 0% |
| | cell collection was not conducted | 108 | 156 | 132 | - |
| N. meningitis | BSA | 44 | 46 | 45 | 82% |
| | saline | | 17 | 12 | 22% |
| | cell collection was not conducted | 48 | 62 | 55 | - |
| E. coli | BSA | 154 | 142 | 148 | 80% |
| | saline | 106 | 117 | 111.5 | 60% |
| | cell collection was not conducted | 176 | 194 | 185 | - |

The results of Table 3 demonstrated that cells of Corynebacterium renale, which is a gram-positive bacillus, could not be collected by physiological saline, but could be collected by adding BSA. On the other hand, although cells of Neisseria meningitis, which is a gram-negative coccus, and Escherichia coli, which is a gram-negative bacillus, could be collected even if BSA was not added, the cell collection efficiency was improved by adding BSA.

Table 4 shows the results of PCR.

**[Table 4]**

| cell | cell collection | Cq value | | |
|---|---|---|---|---|
| | | test 1 | test 2 | average |
| C. renale | BSA | 33.11 | 32.15 | 32.63 |
| | saline | 38.22 | 40.03 | 39.125 |
| | cell collection was not conducted | 35.42 | NT | 35.42 |
| N. meningitis | BSA | 28.39 | 28.65 | 28.52 |
| | saline | 32.91 | 31.17 | 32.04 |
| | cell collection was not conducted | 30.16 | NT | 30.16 |
| E. coli | BSA | 32.92 | 30.03 | 31.475 |
| | saline | 30.05 | 34.27 | 32.16 |
| | cell collection was not conducted | 34.01 | NT | 34.01 |

The results of Table 4 demonstrated that in also PCR, in Corynebacterium renale, which is a gram-positive bacillus, when BSA was added, the Cq value decreased as compared to that obtained when physiological saline was added. On the other hand, in Neisseria meningitis, which is a gram-negative coccus, and Escherichia coli, which is a gram-negative bacillus, when BSA was added, the Cq value slightly decreased as compared to those obtained when physiological saline was added and when cell collection was not conducted.

### Example 3: Cell collection effect according to type of protein

Staphylococcus aureus (S. aureus) cells precultured using a sheep blood agar medium (trade name: Nissui Plate Sheep Blood Agar, manufactured by Nissui Pharmaceutical Co., Ltd.) were suspended in physiological saline to prepare a bacterial suspension with a turbidity MacFarland of 1.0. The bacterial suspension was then diluted 10³-fold to prepare a diluted bacterial liquid, and 0.1 mL of the diluted bacterial liquid was added to 24.9 mL of physiological saline. To this added were 350 µL of 30% (w/v) BSA solution (fatty acid free, manufactured by FUJIFILM Wako Pure Chemical Corporation) and gelatin or milk protein (trade name: Block Ace, manufactured by DS Pharma Biomedical Co., Ltd.). The solutions were stirred, followed by centrifugation (20,000 G, 15 minutes, 10°C), and the supernatant was removed to obtain 1 mL of each test liquid. These test liquids were diluted 10-fold, and the resulting bacterial liquids were each inoculated on a sheep blood agar medium (the same as above), followed by culturing overnight (37°C, aerobic condition). As a comparison control not performing cell collection, the bacterial suspension was diluted 10⁶-fold, and 100 µL thereof was inoculated on a sheep blood agar medium (the same as above).

DNA was extracted from the test liquid using a bacterial DNA extraction kit (manufactured by Mitsui Chemicals, Inc.), and real time PCR was conducted using Yeast-made Taq DNA polymerase (manufactured by Mitsui Chemicals, Inc.), forward primer: agagtttgatcMtggctcag (SEQ ID NO: 1), reverse primer: ctttacgcccaRtRaWtccg (SEQ ID NO: 2), and probe: 6FAM-tNttaccgcggctgctggcacg-BHQ (SEQ ID NO: 3). The reaction was carried out at 95°C for 5 seconds followed by 45 cycles with 95°C for 5 seconds and 60°C for 30 seconds as one cycle.

Table 5 shows the results of the culture test.

**[Table 5]**

| cell | cell collection | number of colony | | | recovery rate |
|---|---|---|---|---|---|
| | | test 1 | test 2 | average | |
| S. aureus | BSA | 30 | 56 | 43 | 110% |
| | Blockace | 47 | 47 | 47 | 121% |
| | gelatin | 26 | 37 | 31.5 | 81% |
| | saline | 3 | 3 | 3 | 8% |
| | cell collection was not conducted | 28 | 50 | 39 | - |

The results of Table 5 demonstrate that in BSA alone, in milk protein (Block Ace) alone, and in gelatin alone, the number of colonies was close to that obtained when cell collection was not conducted, as compared to that obtained when physiological saline was added.

Table 6 shows the results of PCR.

**[Table 6]**

| cell | cell collection | Cq value | | |
|---|---|---|---|---|
| | | test 1 | test 2 | average |
| S. aureus | BSA | 37.02 | 34.96 | 35.99 |
| | gelatin | 34.14 | 35.88 | 35.01 |
| | saline | 41.75 | 39.91 | 40.83 |
| | cell collection was not conducted | 36.72 | NT | 36.72 |

The results of Table 6 demonstrated that also in PCR, gelatin showed a Cq value equivalent to that in BSA. Accordingly, cells of Staphylococcus aureus, which is a gram-positive bacterium, can be efficiently collected using gelatin as the protein instead of BSA.

### Example 4: Cell collection test using water-insoluble support

Corynebacterium renale (C. renale) and Enterococcus faecalis (E. faecalis) cells precultured using a sheep blood agar medium (trade name: Nissui Plate Sheep Blood Agar, manufactured by Nissui Pharmaceutical Co., Ltd.) were each suspended in physiological saline to prepare bacterial suspensions with a turbidity MacFarland of 1.0. The bacterial suspensions were then each diluted 10³-fold to prepare diluted bacterial liquids, and 0.1 mL of each of the diluted bacterial liquids was added to 24.9 mL of physiological saline. To this added were 350 µL of 30% (w/v) BSA solution and 2.5 µL of 10% (w/v) latex solution as a water-insoluble support. The solutions were stirred, followed by centrifugation (20,000 G, 15 minutes, 10°C), and the supernatant was removed to obtain 1 mL of each test liquid. These test liquids were diluted 10-fold, and 90 µL of each thereof was inoculated on a sheep blood agar medium (the same as above), followed by culturing overnight (37°C, aerobic condition). As a comparison control not performing cell collection, the bacterial suspension was diluted 10⁵-fold, and 100 µL thereof was inoculated on a sheep blood agar medium (the same as above).

DNA was extracted from the test liquid using a bacterial DNA extraction kit (manufactured by Mitsui Chemicals, Inc.), and real time PCR was conducted using Yeast-made Taq DNA polymerase (manufactured by Mitsui Chemicals, Inc.), forward primer: agagtttgatcMtggctcag (SEQ ID NO: 1), reverse primer: ctttacgcccaRtRaWtccg (SEQ ID NO: 2), and probe: 6FAM-tNttaccgcggctgctggcacg-BHQ (SEQ ID NO: 3). The reaction was carried out at 95°C for 5 seconds followed by 45 cycles with 95°C for 5 seconds and 60°C for 30 seconds as one cycle.

Table 7 shows the results of the culture test.

**[Table 7]**

| cell | cell collection | number of colony | | | recovery rate |
|---|---|---|---|---|---|
| | | test 1 | test 2 | average | |
| C. renale | BSA + latex | 119 | 90 | 101 | 97% |
| | saline | 0 | 0 | 0 | 0% |
| | cell collection was not conducted | 126 | 105 | 115.5 | - |
| E. faecalis | BSA + latex | 156 | 167 | 162 | 90% |
| | saline | 17 | 19 | 18 | 10% |
| | cell collection was not conducted | 194 | 207 | 200.5 | - |

The results of Table 7 demonstrated that when BSA and latex were added in combination for collecting cells of Corynebacterium renale, which is a gram-positive bacillus, the recovery rate was 97% of the theoretical value when cell collection was not conducted. In contrast, when physiological saline was added, the recovery rate was 0% of the theoretical value. Accordingly, use of BSA and latex in combination enables to collect the cell of Corynebacterium renale very efficiently.

In addition, when BSA and latex were added in combination for collecting cells of Enterococcus faecalis, which is a gram-positive coccus, the recovery rate was 90% of the theoretical value when cell collection was not conducted. In contrast, when physiological saline was added, the recovery rate was 10% of the theoretical value. Accordingly, it was demonstrated use of BSA and latex in combination enables to collect the cell of Enterococcus faecalis very efficiently.

IN each test, it was found that the precipitate by centrifugation was visualized by adding latex in addition to BSA, and the supernatant was consequently easily removed in any tests.

Table 8 shows the results of PCR.

**[Table 8]**

| cell | cell collection | Cq value | | |
|---|---|---|---|---|
| | | test 1 | test 2 | avarage |
| C. renale | BSA + latex | 35.24 | 34.42 | 34.83 |
| | saline | 41.91 | N/A | 41.91 |
| | cell collection was not conducted | 36.59 | NT | 36.59 |
| E. faecalis | BSA + latex | 34.24 | 34.39 | 34.315 |
| | saline | 42.23 | 42.65 | 42.44 |
| | cell collection was not conducted | 36.86 | NT | 36.86 |

The results of Table 8 demonstrated that also in PCR, when cell collection was conducted by adding BSA and latex in combination, the Cq value was close to the theoretical value when cell collection was not conducted, and the cell collection efficiency was thus sufficient. In contrast, when only physiological saline was added, the Cq value increased, and the cell collection was thus insufficient.

### Example 5: Influence of pH on cell collection

Staphylococcus aureus (S. aureus) cells precultured using a sheep blood agar medium (trade name: Nissui Plate Sheep Blood Agar, manufactured by Nissui Pharmaceutical Co., Ltd.) were suspended in physiological saline to prepare a bacterial suspension with a turbidity MacFarland of 1.0. The bacterial suspension was then diluted 10³-fold to prepare a diluted bacterial liquid, and 0.1 mL of the diluted bacterial liquid was added to 24.9 mL of 0.01 M sodium phosphate buffer solutions having a pH 6.0, pH 7.0, or pH 8.0. To these added was 350 µL of 30% (w/v) BSA solution (bovine serum-derived BSA solution, fatty acid free, manufactured by FUJIFILM Wako Pure Chemical Corporation). The solutions were stirred, followed by centrifugation (20,000 G, 15 minutes, 10°C), and the supernatant was removed to obtain 1 mL of each test liquid. These test liquids were diluted 10-fold, and 10 µL of the resulting bacterial liquids were each inoculated on a sheep blood agar medium (the same as above), followed by culturing overnight (37°C, aerobic condition). As a comparison control not performing cell collection, the bacterial suspension was diluted 10⁶⁻fold with physiological saline, and 100 µL thereof was inoculated on a sheep blood agar medium (the same as above).

DNA was extracted from the test liquid using a bacterial DNA extraction kit (manufactured by Mitsui Chemicals, Inc.), and real time PCR was conducted using Yeast-made Taq DNA polymerase (manufactured by Mitsui Chemicals, Inc.), forward primer: agagtttgatcMtggctcag (SEQ ID NO: 1), reverse primer: ctttacgcccaRtRaWtccg (SEQ ID NO: 2), and probe: 6FAM-tNttaccgcggctgctggcacg-BHQ (SEQ ID NO: 3). The reaction was carried out at 95°C for 5 seconds followed by 45 cycles with 95°C for 5 seconds and 60°C for 30 seconds as one cycle.

Table 9 shows the results of the culture test.

**[Table 9]**

| cell | specimen solution | cell collection | number of colony | | |
|---|---|---|---|---|---|
| | | | test 1 | test 2 | average |
| S. aureus | sodium phosphate buffer (pH 6.0) | in the presence of BSA | 35 | 38 | 36.5 |
| | | in the absence of BSA | 4 | 5 | 4.5 |
| | sodium phosphate buffer (pH 7.0) | in the presence of BSA | 65 | 36 | 50.5 |
| | | in the absence of BSA | 6 | 8 | 7 |
| | sodium phosphate buffer (pH 8.0) | in the presence of BSA | 30 | 33 | 31.5 |
| | | in the absence of BSA | 6 | 6 | 6 |
| | saline | cell collection was not conducted | 34 | 21 | 27.5 |

The pH value after addition of BSA was measured three times. Table 10 shows the measurement results together with the averages thereof.

**[Table 10]**

| specimen solution | pH value after BSA addition | | | average |
|---|---|---|---|---|
| sodium phosphate buffer (pH 6.0) | 6.3 | 6.3 | 6.2 | 6.3 |
| sodium phosphate buffer (pH 7.0) | 7.2 | 7.3 | 7.1 | 7.2 |
| sodium phosphate buffer (pH 8.0) | 7.9 | 8.0 | 8.0 | 8.0 |

Table 9 demonstrated that when BSA was added, in any of pH regions of pH 6.0, pH 7.0, and pH 8.0, cells of Staphylococcus aureus, which is a gram-positive coccus, could be collected and the cells could be cultured. In addition, the results of Table 10 demonstrated that almost no fluctuation was observed in pH after addition of BSA.

Table 11 shows the results of PCR.

**[Table 11]**

| cell | specimen solution | cell collection | number of colony | | |
|---|---|---|---|---|---|
| | | | test 1 | test 2 | average |
| S. aureus | sodium phosphate buffer (pH 6.0) | in the presence of BSA | 30.49 | 30.72 | 30.605 |
| | | in the absence of BSA | 40.42 | 37 | 38.71 |
| | sodium phosphate buffer (pH 7.0) | in the presence of BSA | 31.7 | 30.81 | 31.255 |
| | | in the absence of BSA | 41.23 | 35.42 | 38.325 |
| | sodium phosphate buffer (pH 8.0) | in the presence of BSA | 31.44 | 30.83 | 31.135 |
| | | in the absence of BSA | 38.54 | 35.86 | 37.2 |
| | saline | cell collection was not conducted | 30.11 | NT | 30.11 |

The results of Table 11 demonstrated that when BSA was added to each of sodium phosphate buffer solutions having pH 6.0, pH 7.0, or pH 8.0, the Cq values were close to that obtained when cell collection was not conducted. In contrast, when the specimen solution not containing BSA was added, the Cq value was shown to be higher than that obtained when cell collection was not conducted, and the cell collection was therefore insufficient.

### Example 6: Result of Clostridium sporogenes

Clostridium sporogenes (C. sporogenes) cells precultured using a sheep blood agar medium (trade name: Nissui Plate Sheep Blood Agar, manufactured by Nissui Pharmaceutical Co., Ltd.) were suspended in physiological saline to prepare a bacterial suspension with a turbidity MacFarland of 1.0. The bacterial suspension was then diluted 10³-fold to prepare a diluted bacterial liquid, and 0.1 mL of the diluted bacterial liquid was added to 24.9 mL of physiological saline. To this added was 350 µL of 30% (w/v) BSA solution (bovine serum-derived BSA solution, fatty acid free, manufactured by FUJIFILM Wako Pure Chemical Corporation). The solutions were stirred, followed by centrifugation (20,000 G, 15 minutes, 10°C), and the supernatant was removed to obtain 1 mL of a test liquid. This test liquid was diluted 10-fold, and 10 µL of the resulting bacterial liquid was inoculated on a sheep blood agar medium (the same as above), followed by culturing overnight (37°C) using Anaeromate-P "Nissui" (manufactured by Nissui Pharmaceutical Co., Ltd.). As a comparison control not performing cell collection, the bacterial suspension was diluted 10⁶⁻fold with physiological saline, and 100 µL thereof was inoculated on a sheep blood agar medium (the same as above).

DNA was extracted from the test liquid using a bacterial DNA extraction kit (manufactured by Mitsui Chemicals, Inc.), and real time PCR was conducted using Yeast-made Taq DNA polymerase (manufactured by Mitsui Chemicals, Inc.), forward primer: agagtttgatcMtggctcag (SEQ ID NO: 1), reverse primer: ctttacgcccaRtRaWtccg (SEQ ID NO: 2), and probe: 6FAM-tNttaccgcggctgctggcacg-BHQ (SEQ ID NO: 3). The reaction was carried out at 95°C for 5 seconds followed by 45 cycles with 95°C for 5 seconds and 60°C for 30 seconds as one cycle.

Table 12 shows the results of the culture test.

**[Table 12]**

| cell | cell collection | number of colony | | | recovery rate |
|---|---|---|---|---|---|
| | | test 1 | test 2 | average | |
| C. sporogenes | BSA | 49 | 73 | 61 | 151% |
| | saline | 26 | 20 | 23 | 57% |
| | cell collection was not conducted | 47 | 34 | 40.5 | - |

The results of Table 12 demonstrated that when BSA was added in cell collection of Clostridium sporogenes, which is a gram-positive bacillus, the recovery rate was 151% of the theoretical value when cell collection was not conducted. In contrast, when physiological saline was added, the recovery rate was 57%. Accordingly, use of BSA enables to collect the cell Clostridium sporogenes very efficiently.

Table 13 shows the results of PCR.

**[Table 13]**

| cell | cell collection | | Cq value | |
|---|---|---|---|---|
| | | test 1 | test 2 | avarage |
| C. sporogenes | BSA | 36.62 | 34.28 | 35.45 |
| | saline | 40.39 | 35.97 | 38.18 |
| | cell collection was not conducted | 32.39 | NT | 32.39 |

The results of Table 13 demonstrated that also in PCR, when cell collection was conducted by adding BSA, the Cq value was close to the theoretical value when cell collection was not conducted, and the cell collection efficiency was thus sufficient. In contrast, when only physiological saline was added, the Cq value increased, and the cell collection was thus insufficient.

### Example 7: Influence of pH in acidic region

Staphylococcus aureus (S. aureus) cells precultured using a sheep blood agar medium (trade name: Nissui Plate Sheep Blood Agar, manufactured by Nissui Pharmaceutical Co., Ltd.) were suspended in physiological saline to prepare a bacterial suspension with a turbidity MacFarland of 1.0. The bacterial suspension was then diluted 10³-fold to prepare a diluted bacterial liquid, and 0.1 mL of the diluted bacterial liquid was added to 24.9 mL of 0.01 M sodium citrate buffer solution having a pH of 4.3. To this added was 350 µL of 30% (w/v) BSA solution (bovine serum-derived BSA solution, fatty acid free, manufactured by FUJIFILM Wako Pure Chemical Corporation). The solutions were stirred, followed by centrifugation (20,000 G, 15 minutes, 10°C), and the supernatant was removed to obtain 1 mL of a test liquid. This test liquid was diluted 10-fold, and 10 µL of the resulting bacterial liquid was inoculated on a sheep blood agar medium (the same as above), followed by culturing overnight (37°C, aerobic condition). As a comparison control not performing cell collection, the bacterial suspension was diluted 10⁶⁻fold with physiological saline, and 100 µL thereof was inoculated on a sheep blood agar medium (the same as above).

DNA was extracted from the test liquid using a bacterial DNA extraction kit (manufactured by Mitsui Chemicals, Inc.), and real time PCR was conducted using Yeast-made Taq DNA polymerase (manufactured by Mitsui Chemicals, Inc.), forward primer: agagtttgatcMtggctcag (SEQ ID NO: 1), reverse primer: ctttacgcccaRtRaWtccg (SEQ ID NO: 2), and probe: 6FAM-tNttaccgcggctgctggcacg-BHQ (SEQ ID NO: 3). The reaction was carried out at 95°C for 5 seconds followed by 45 cycles with 95°C for 5 seconds and 60°C for 30 seconds as one cycle.

Table 14 shows the results of the culture test.

**[Table 14]**

| cell | specimen solution | cell collection | number of colony | | |
|---|---|---|---|---|---|
| | | | test 1 | test 2 | average |
| S. aureus | sodium citrate buffer (pH 4.3) | in the presence of BSA | 33 | 27 | 30 |
| | | in the absence of BSA | 34 | 26 | 30 |
| | saline | cell collection was not conducted | 32 | 28 | 30 |

The pH value after addition of BSA was measured three times. Table 15 shows the measurement results together with the averages thereof.

**[Table 15]**

| specimen solution | pH value after BSA addition | | | average |
|---|---|---|---|---|
| sodium citrate buffer (pH 4.3) | 4.7 | 4.6 | 4.6 | 4.6 |

Table 14 demonstrated that when the pH region was pH 4.3, cells of Staphylococcus aureus, which is a gram-positive coccus, could be collected regardless of whether BSA was added or not. The results of Table 15 demonstrated that almost no fluctuation was observed in pH after addition of BSA.

Table 16 shows the results of PCR.

**[Table 16]**

| cell | specimen solution | cell collection | Cq value | | |
|---|---|---|---|---|---|
| | | | test 1 | test 2 | average |
| S. aureus | sodium citrate buffer (pH 4.3) | in the presence of BSA | 29.82 | 30.79 | 30.31 |
| | | in the absence of BSA | 33.20 | 31.49 | 32.35 |
| | saline | cell collection was not conducted | 31.56 | NT | 31.56 |

The results of Table 16 demonstrated that when a sodium citrate buffer solution having a pH of 4.3 was used, the Cq value was close to that when cell collection was not conducted, regardless of whether BSA was added or not.

### Example 8: Collection of cells of Candida albicans

Two pieces of BioBall 550 (trade name: BioBall 550, manufactured by bioMerieus Japan Ltd.) were added to 25 mL of physiological saline to prepare a bacterial suspension equivalent to 1,100 CFU/25 mL. To this added were 350 µL of 30% (w/v) BSA solution (bovine serum-derived BSA solution, fatty acid free, manufactured by FUJIFILM Wako Pure Chemical Corporation) and 2.5 µL of 10% (w/v) latex solution as a water-insoluble support. The solutions were stirred, followed by centrifugation (20,000 G, 15 minutes, 10°C). The supernatant was removed to obtain 1 mL of a test liquid, and 100 µL of this test liquid was inoculated on a sheep blood agar medium (trade name: Nissui Plate Sheep Blood Agar, manufactured by Nissui Pharmaceutical Co., Ltd.), followed by culturing overnight (37°C, aerobic condition).

Table 17 shows the results of the culture test.

**[Table 17]**

| cell | cell collection | number of colony | | | recovery rate |
|---|---|---|---|---|---|
| | | test 1 | test 2 | average | |
| C. albicans | BSA | 93 | 84 | 88.56 | 80.5% |
| | saline | 68 | 77 | 72.5 | 65.9% |
| | theoretical value | 110 | | | - |

The results of Table 17 demonstrated that when BSA and latex were added in cell collection of Candida albicans, which is a fungus showing gram-positive, the recovery rate was 80.5% of the theoretical value, which was better than 65.9% when physiological saline was added.

## Claims

1. A method for collecting cells of one or more microorganisms selected from the group consisting of gram-positive bacteria (excluding mycoplasma), gram-negative bacteria, and fungi in a specimen, the method comprising adding one or more proteins selected from the group consisting of albumin, casein, hydrolyzed casein, milk protein, and gelatin to the specimen and collecting a resulting flocculate.

2. The method according to claim 1, wherein the method collects cells of gram-positive bacteria (excluding mycoplasma) and/or gram-negative bacteria.

3. The method according to claim 1 or 2, wherein the specimen after addition of the protein has a pH of above 5.0 and 11.0 or less.

4. The method according to any one of claims 1 to 3, wherein the specimen is a liquid specimen selected from the group consisting of sterilized water, physiological saline, a liquid culture medium, a biological sample, a culture supernatant, a buffer solution, and a Ringer's solution.

5. The method according to any one of claims 1 to 4, wherein the method collects cells of gram-positive bacteria.

6. The method according to any one of claims 1 to 5, wherein the gram-positive bacteria are one or more selected from the group consisting of Staphylococcus, Enterococcus, Streptococcus, Clostridium, Corynebacterium, and Bacillus.

7. The method according to any one of claims 1 to 5, wherein the gram-positive bacteria are one or more selected from the group consisting of Staphylococcus aureus, Bacillus subtilis, and Clostridium sporogenes.

8. The method according to any one of claims 1 to 7, wherein a water-insoluble support is further added to the specimen.

9. A method for measuring one or more microorganisms selected from the group consisting of gram-positive bacteria, gram-negative bacteria, and fungi, the method comprising subjecting cells of the one or more microorganisms selected from the group consisting of gram-positive bacteria, gram-negative bacteria, and fungi collected by the method according to any one of claims 1 to 8 to a polymerase chain reaction.

10. A method for measuring one or more microorganisms selected from the group consisting of gram-positive bacteria, gram-negative bacteria, and fungi, the method comprising enrichment of cells of the one or more microorganisms selected from the group consisting of gram-positive bacteria, gram-negative bacteria, and fungi collected by the method according to any one of claims 1 to 8.
